# EUROPÄISCHE PATENTSCHRIFT

(11) **EP 2 503 322 B1**
(45) Veröffentlichungstag und Bekanntmachung des Hinweises auf die Patenterteilung: **09.03.2016**
(21) Anmeldenummer: 11159845.4
(22) Anmeldetag: 25.03.2011
(51) Int. Cl.: G01N 21/3563, G01N 21/85, G01N 21/95, G01N 21/15, A61J 7/02, B07C 5/34, G01N 33/15, G01N 25/72, B65B 37/04, B65B 57/14, B07C 5/36, B65B 5/10

(54) **Vorrichtung zum Überprüfen von kleinen pharmazeutischen Produkten**
Device for inspecting small pharmaceutical products
Dispositif de vérification de petits produits pharmaceutiques

(43) Veröffentlichungstag der Anmeldung: 26.09.2012
(73) Patentinhaber: Gertitschke, Detlev, 88471 Laupheim (DE)
(72) Erfinder: Gertitschke, Detlev, 88471 Laupheim (DE)
(74) Vertreter: Westphal, Mussgnug & Partner Patentanwälte mbB

(56) Entgegenhaltungen:
- WO-A2-2009/019698
- DE-C1- 4 312 550
- US-A- 5 027 938
- US-A- 5 638 417
- US-A- 5 884 806
- US-B1- 6 324 253

## Beschreibung

Die vorliegende Erfindung betrifft eine Vorrichtung zum Überprüfen von vereinzelten Tabletten oder Kapseln.

Eine wichtige Funktion beim Abfüllen von Tabletten oder Kapseln in Flaschen oder andere Behälter ist das Zählen und die Ausscheidung fehlerhafter Tabletten oder Kapseln. Auf dem Markt befindliche Systeme arbeiten mit unterschiedlichen Technologien.

Farbkameras können mit entsprechender Bildauswertung falschfarbige oder gebrochene Tabletten oder Kapseln detektieren. Hierfür ist aufgrund der kontinuierlichen Bewegung der vorbei geführten Tabletten oder Kapseln allerdings eine hoch komplexe Auswertesoftware erforderlich. Außerdem ist ein hoher Beleuchtungsaufwand notwendig, um ausreichend scharfe Bildinhalte zu erzielen.

Kapazitive Sensoren können eingesetzt werden, um berührungslos die Masse der vorbei geführten Tabletten oder Kapseln zu berechnen und daraus Rückschlüsse auf die Unversehrtheit der Tablette oder Kapsel zu ziehen. Allerdings sind kapazitive Sensoren empfindlich gegenüber Schwingungen, elektrischen Störfeldern und dem Feuchtigkeitsgehalt der Tabletten oder Kapseln.

US 5 638 417 beschreibt eine Vorrichtung zum Zählen von Tabletten unabhängig von ihrer Größe, wobei diese durch eine Detektoreinrichtung fallen, die aus einer Infrarot-LED und einem entsprechenden Detektor besteht, und dabei ein entsprechendes Zählsignal erzeugen.

Aus US 6 324 253 B1 ist eine Vorrichtung bekannt, bei der Tabletten, die sich bereits in einer Verpackungsbahn befinden, durch Röntgen- oder Infrarotstrahlung erkannt werden. Dadurch kann kontrolliert werden, ob den Verpackungsfächern die richtigen Tabletten zugeführt wurden.

DE 43 12 550 C1, die den nächstkommenden Stand der Technik darstellt, offenbart eine Vorrichtung, die dazu geeignet ist, die Form und Fläche der Tabletten zu berechnen. Hierzu fallen die Tabletten in einem Schacht durch eine Messebene, die aus einer Anordnung aus mehreren Infrarot-LEDs und den entsprechenden Sensorleisten besteht. Beim Durchtritt der Tablette erzeugt diese einen Schatten vor den LEDs, der von den Sensoren wahrgenommen wird. Durch zeitliche Aneinanderreihung dieser Schattenbilder kann ein Bild der gesamten Tablette gewonnen werden. Außerdem ist es möglich, unter Kenntnis der Fallgeschwindigkeit die Fläche der Tablette zu berechnen. Durch einen Soll-/ Istwert-Vergleich können beschädigte Tabletten ausgeschieden werden.

Das Verfahren gemäß WO 2009/019698 A kann dazu eingesetzt werden, gefälschte Tabletten von den Originalen zu unterscheiden. Die Tabletten werden einer Wärmebehandlung unterzogen, um anschließend ein thermografisches Bild zu erzeugen, das dem einer Wärmebildkamera ähnlich ist. Dieses Bild kann dann zur Erkennung der Fälschung mit einem Referenzbild einer Originaltablette verglichen werden.

Der vorliegenden Erfindung liegt die Aufgabe zugrunde, eine Vorrichtung zum Überprüfen von Tabletten oder Kapseln zu schaffen, die sehr robust ist, einfach aufgebaut ist und bei verschiedenen Produkttypen und -formaten angewendet werden kann.

Diese Aufgabe wird durch die Merkmale des Anspruchs 1 gelöst.

Erfindungsgemäß umfasst die Vorrichtung zum Überprüfen von kleinen pharmazeutischen Produkten eine Führungseinrichtung für vereinzelte Tabletten oder Kapseln, die einen vorbestimmten Tablettenpfad definiert, ein im Bereich der Führungseinrichtung angeordnetes und als Wärmeplatte ausgebildetes Wärmeelement, einen dem Wärmeelement gegenüberliegenden IR-Sensor zur Detektion von Strahlung im Infrarotbereich, wobei der Tablettenpfad zwischen dem Wärmeelement und dem IR-Sensor verläuft, und eine Auswerteeinheit, die dazu geeignet ist, Ist-Messwerte des IR-Sensors mit mindestens einem vorbestimmten Soll-Messwert zu vergleichen.

Die Ist-Messwerte des IR-Sensors sind Temperaturmesswerte.

Mit dieser Anordnung können vereinzelte Tabletten oder Kapseln schnell und sicher hinsichtlich ihrer Farbe, Form und Unversehrtheit untersucht werden, und das Wärmeelement gewährleistet eine definierte Abstrahlung und beansprucht gleichzeitig wenig Platz. Die Vorrichtung ist äußerst robust gegenüber äußeren Einflüssen und erlaubt den Einsatz bei verschiedenen Tablettenformen und -größen.

Das Wärmeelement ist dabei vorzugsweise von der Führungseinrichtung entkoppelt. Auf diese Weise wird die Zuverlässigkeit der Vorrichtung erhöht.

Zur Optimierung der Messergebnisse ist das Wärmeelement vorzugsweise bezüglich seiner Temperatur einstellbar.

Zur Erzeugung einer gleichmäßigen Temperatur des Wärmeelements und einer einfachen Erwärmung desselben ist das Wärmeelement vorzugsweise aus Metall, mehr bevorzugt aus Aluminium, gebildet.

Da glatte Metalle in der Regel einen relativ geringen Emissionswert besitzen, weist das Wärmeelement auf einer dem Tablettenpfad zugewandten Fläche vorzugsweise eine nichtmetallische Beschichtung mit hohem Emissionswert auf. Dies hat den zusätzlichen Vorteil, dass der Emissionswert von Nichtmetallen kaum von der Temperatur und der Wellenlange abhängt.

Um eine Schädigung der Tabletten oder Kapseln aufgrund der hohen Temperatur des Wärmeelements zu verhindern, kann zwischen Wärmeelement und Tablettenpfad eine IR-durchlässige Platte angeordnet sein, vorzugsweise aus IR-durchlässigem Glas.

Zur exakten Einstellung des Messfeldes und einer möglichen Anpassung an unterschiedliche Tablettengrößen ist vorzugsweise zwischen Wärmeelement und Tablettenpfad eine Messfeldblende angeordnet.

Vorzugsweise weist der IR-Sensor eine Vorsatzlinse zur Einstellung der Messfeldgröße auf.

Der Abstand zwischen IR-Sensor und Messfeld liegt je nach Produktform vorzugsweise im Bereich von 20 bis 50 mm.

Das zugehörige Messfeld weist je nach Produktform und -art eine variable Größe von 25 bis 1000 mm² auf.

Die vom IR-Sensor gemessenen Ist-Messwerte sind Temperaturmesswerte.

Diese Temperaturmesswerte werden vom IR-Sensor vorzugsweise über die IR-Strahlung ermittelt, welche vom gesamten Bereich des Messfeldes empfangen werden, und sind somit Temperaturdurchschnittswerte.

In einer bevorzugten Ausführungsform misst der IR-Sensor durchgängig eine Temperaturkurve, welche nach vorbestimmten Kriterien mit einer entsprechenden SollTemperaturkurve verglichen werden kann.

In einer bevorzugten Ausführungsform ist die Führungseinrichtung eine Vibrationsrinne. Ebenso kann die Führungseinrichtung als Produktrutsche ohne Vibration ausgebildet sein.

Weitere Vorteile und Merkmale der vorliegenden Erfindung ergeben sich aus der nachfolgenden Beschreibung unter Bezugnahme auf die Zeichnungen.
Fig. 1 ist eine schematische Ansicht sämtlicher Komponenten eines bevorzugten Ausführungsbeispiels einer erfindungsgemäßen Vorrichtung zur Überprüfung von kleinen pharmazeutischen Produkten;
Fig. 2 ist eine schematische Frontansicht eines Teils einer ersten bevorzugten Ausführungsform der erfindungsgemäßen Vorrichtung zur Überprüfung von kleinen pharmazeutischen Produkten;
Fig. 3 ist eine schematische Seitenansicht eines Teils einer zweiten bevorzugten Ausführungsform der erfindungsgemäßen Vorrichtung zum Überprüfen von kleinen pharmazeutischen Produkten.
Fig. 4 ist eine schematische Skizze, die das erfindungsgemäße Messprinzip veranschaulicht;
Fig.5 ist ein Graph, der beispielhaft den Zusammenhang zwischen Tablettendurchmesser und überprüften Tabletten pro Sekunde darstellt;
Fig. 6 ist eine schematische Ansicht eines möglichen Verlaufs der Ist-Messwerte des IR-Sensors;
Fig. 7 bis 9 sind schematische Graphen, welche theoretisch zu erwartende Messergebnisse des IR-Sensors unter verschiedenen Bedingungen zeigen; und
Fig. 10 ist ein tatsächlich aufgenommener Graph von Ist-Messwerten des IR-Sensors für eine ganze und eine abgebrochene Tablette.

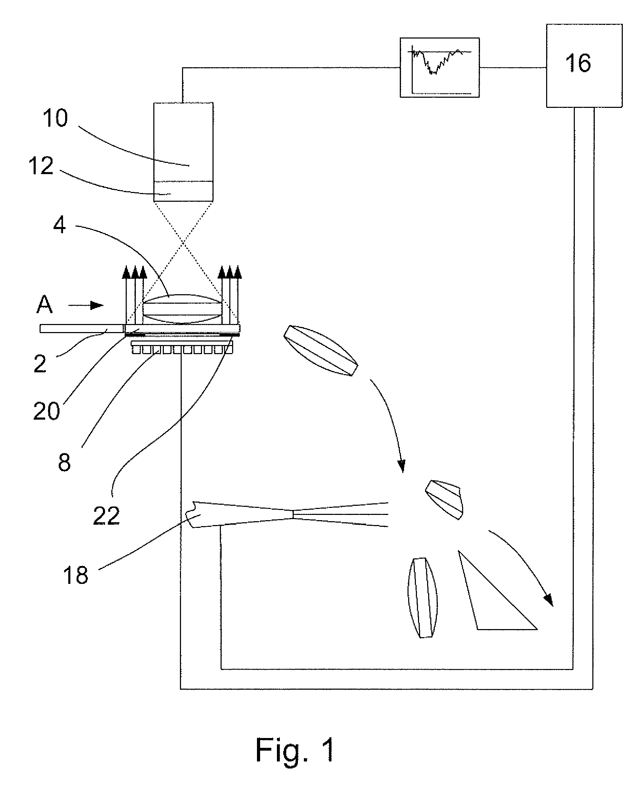

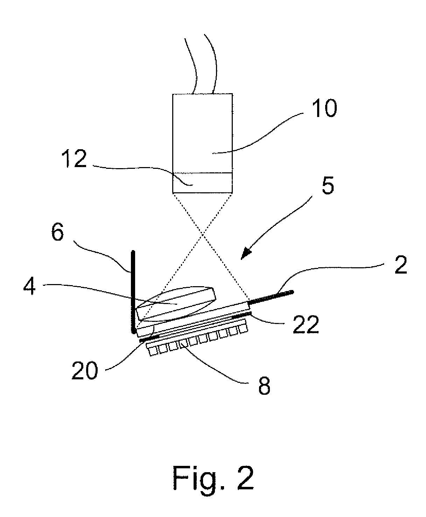

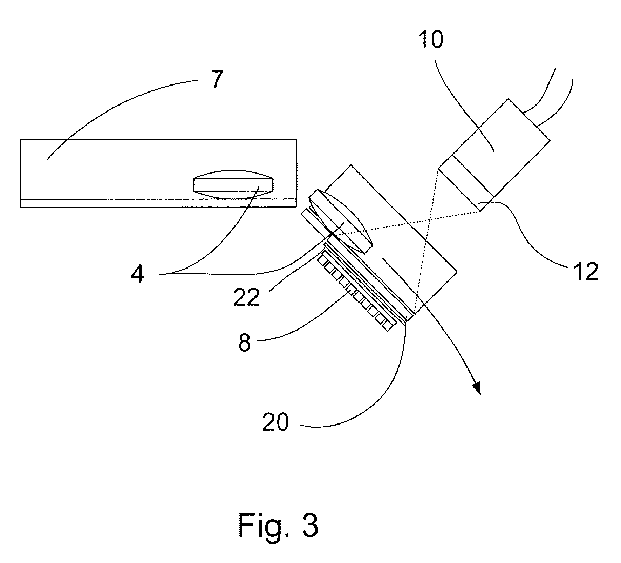

In Fig. 1 sind die Komponenten eines bevorzugten Ausführungsbeispiels einer erfindungsgemäßen Vorrichtung zum Überprüfen von kleinen pharmazeutischen Produkten (Tabletten oder Kapseln) schematisch dargestellt. Im Folgenden wird an Stelle des Begriffs "kleines pharmazeutisches Produkt" zur Vereinfachung der Begriff "Tablette" verwendet. Die Vorrichtung umfasst zunächst eine Führungseinrichtung 2 für vereinzelte Tabletten 4, die einen vorbestimmten Tablettenpfad A definiert, entlang dem die Tabletten 4 vorwärts bewegt werden. Die Führungseinrichtung 2 kann beispielsweise, wie in Fig. 2 dargestellt, als Vibrationsrinne ausgestaltet sein. In diesem Fall kann die Führungseinrichtung 2 beispielsweise V-förmige Rillen 5 mit einem mehr oder weniger senkrechten Wandabschnitt 6 für eine exakte Führung der Tabletten 4 aufweisen, wie in Fig. 2 dargestellt ist. Alternativ kann die Führungseinrichtung 2 als nicht vibrierende Produktrutsche ausgestaltet sein, der die Produkte von einer Transportvorrichtung 7, etwa einer Vibrationsrinne, vereinzelt zugeführt werden, wie in Fig. 3 dargestellt ist.

Die Vorrichtung zum Überprüfen von Tabletten weist ein Wärmeelement 8 auf, welches gleichmäßig auf eine vorbestimmte Temperatur aufgeheizt wird. Die Temperatur des Wärmeelements beträgt vorzugsweise 40 bis 80°C, mehr bevorzugt 50 bis 70°C. Das Wärmeelement 8 besteht vorzugsweise aus einem gut Wärme leitenden Metall, beispielsweise aus Aluminium, und wird direkt über einen Widerstandsdraht beheizt. Alternativ kann das Wärmeelement 8 auch berührungslos über Induktion oder über Infrarotstrahlung beheizt werden.

Das Wärmeelement 8 ist dabei auf eine für die entsprechende Anwendung geeignete Temperatur einstellbar und ist vorzugsweise von der Führungseinrichtung 2 entkoppelt gelagert und damit keinen Schwingungen ausgesetzt. Erfindungsgemäß ist das Wärmeelement 8 als Wärmeplatte ausgebildet.

Wichtig ist hierbei, dass das Wärmeelement 8 eine möglichst gleichmäßige Temperatur besitzt und somit eine möglichst gleichmäßige IR-Strahlung über ihre Fläche in Richtung des Tablettenpfades A ausstrahlt. Das Wärmeelement 8 ist in jedem Fall im Bereich der Führungseinrichtung 2 angeordnet.

Da im Rahmen der Erfindung die IR-Strahlung des Wärmeelements 8 möglichst hoch sein soll, Metalle aber einen relativ geringen Emissionswert ε besitzen, ist das Wärmeelement 8 auf seiner dem Tablettenpfad A zugewandten Fläche vorzugsweise mit einer nicht metallischen Beschichtung mit hohem Emissionswert bezogen. Die Beschichtung kann beispielsweise aus matt schwarzem, hitzebeständigen Lack bestehen und weist vorzugsweise einen Emissionswert ε von > 0,9, besonders bevorzugt > 0,95 auf.

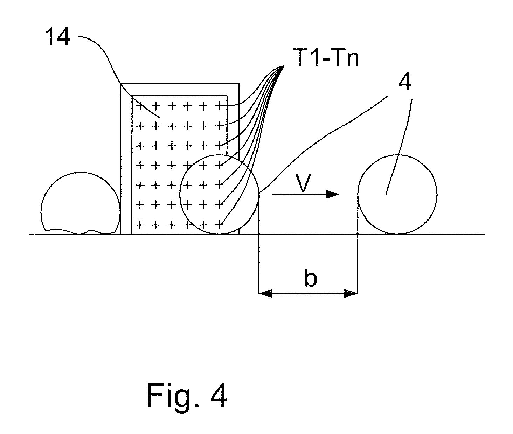

Gegenüber dem Wärmeelement 8 ist ein Infrarotsensor (IR-Sensor) 10 zur Detektion von Strahlung im Infrarotbereich angeordnet, wobei der Tablettenpfad A zwischen dem Wärmeelement 8 und dem IR-Sensor 10 verläuft. Der IR-Sensor 10 weist vorzugsweise eine Vorsatzlinse 12 auf, wodurch er die Größe eines zweidimensionalen Messfeldes 14 entlang des Tablettenpfades A einstellt (siehe insbesondere Fig. 4). Der IR-Sensor 10 weist in bevorzugten Ausführungsformen einen Abstand vom Messfeld 14 von 20 bis 50 mm auf, und das Messfeld 14 kann abhängig von der jeweiligen Anwendung eine variable Größe von 25 bis 1000 mm² aufweisen. Der IR-Sensor 10 kann mit Staubschutz und einer Freiblaseinrichtung (nicht dargestellt) ausgerüstet sein. Der IR-Sensor 10 besitzt vorzugsweise eine äußerst kurze Ansprechzeit von wenigen Millisekunden. Falls nötig, kann der Bereich zwischen IR-Sensor 10 und Messfeld 14 zur Abschottung vor Fremdstrahlung von einem Gehäuse umgeben sein. Versuche haben jedoch gezeigt, dass die Funktion der Vorrichtung durch Fremdstrahlung auch ohne Gehäuse nahezu nicht beeinträchtigt wird.

Hintergrund für die Messung des IR-Sensors 10 ist, dass jeder Körper abhängig von seiner Temperatur- und Materialbeschaffenheit sowie seiner Farbe IR-Strahlung unterschiedlicher Intensität und Wellenlänge aussendet. Der IR-Sensor 10 ist nun in der Lage, Ist-Messwerte der über den gesamten Bereich des Messfeldes 14 in Richtung des IR-Sensors 10 abgestrahlten IR-Strahlung aufzunehmen und daraus zu jedem beliebigen Zeitpunkt eine Durchschnittstemperatur zu ermitteln.

Wie aus Fig. 4 hervorgeht, befinden sich im Messfeld 14 zu jedem Zeitpunkt eine unendliche Anzahl strahlender Punkte mit einer vorbestimmten Temperatur T1 bis Tn. Bewegt sich nun eine "kalte" Tablette 4 (in der Regel 20-22°C) durch das Messfeld 14, so wird das Wärmelement 8 von der Tablette 4 teilweise abgedeckt und es ergibt sich ein deutlich unterschiedlicher Ist-Messwert des IR-Sensors 10.

Eine in Fig. 1 dargestellte Auswerteeinheit 16 ist darauf ausgerichtet, die Ist-Messwerte des IR-Sensors 10 mit mindestens einem vorbestimmten Soll-Messwert zu vergleichen. Hierauf wird weiter unten noch weiter eingegangen.

Die Auswerteeinheit 16 kann außerdem veranlassen, dass als schlecht klassifizierte Tabletten 4 mittels einer Ausscheidevorrichtung 18, in Fig. 1 beispielhaft als Ausblasdüse dargestellt, ausgeschieden werden.

Aufgrund der großen Wärme des Wärmeelements 8 ist es vorteilhaft, wenn zwischen dem Wärmeelement 8 und dem Tablettenpfad A eine IR-durchlässige, möglichst schlecht Wärme leitende Platte 20 angeordnet ist. Diese kann beispielsweise aus IR-durchlässigem Glas bestehen. Die IR-durchlässige Platte 20 kann, wie in Fig. 2 und 3 dargestellt, Teil der Führungseinrichtung 2 für die Tabletten 4 sein, und zwar derart, dass die Tabletten 4 unmittelbar über die IR-durchlässige Platte 20 bewegt werden bzw. gleiten.

Zur exakten Anpassung des Messfeldes 14 ist es vorteilhaft, wenn zwischen Wärmeelement 8 und dem Tablettenpfad A, besonders bevorzugt zwischen Wärmeelement 8 und der IR-durchlässigen Platte 20, eine Messfeldblende 22 angeordnet ist. Durch Einstellen der Messfeldblende 22 kann die Vorrichtung auf verschiedene Größen und Formen von zu überprüfenden Tabletten 4 optimiert werden, falls dies notwendig ist.

Der Ablauf der Überprüfungsmessung wird nun unter Bezugnahme auf Fig. 1 und 4 nochmals zusammenfassend beschrieben. Die Tabletten 4 werden mit vorzugsweise annähernd gleichmäßiger Fördergeschwindigkeit v und bevorzugt auch annähernd gleichem Abstand b sowie nahezu konstanter Temperatur (vorzugsweise im Bereich zwischen 20 und 22°C) entlang der Führungseinrichtung 2 vorwärts bewegt bzw. gleiten an dieser entlang. Dabei bewegen sich die Tabletten 4 auch durch das Messfeld 14, das gegebenenfalls durch die Messfeldblende 22 definiert wird. Durchläuft die Tablette 4 das Messfeld 14, so schattet die kalte Tablette 4 einen Teil des Wärmeelements 8 ab. Dies wird durch den IR-Sensor 10 detektiert, und das Ist-Messergebnis des IR-Sensors 10 wird in der Auswerteeinheit 16 mit einem eingelernten Soll-Messwert verglichen. Bei großer Differenz zwischen Ist-Messwert und Soll-Messwert wird ein Fehlersignal ausgelöst, das zur Aktivierung der Ausscheidevorrichtung 18 führt.

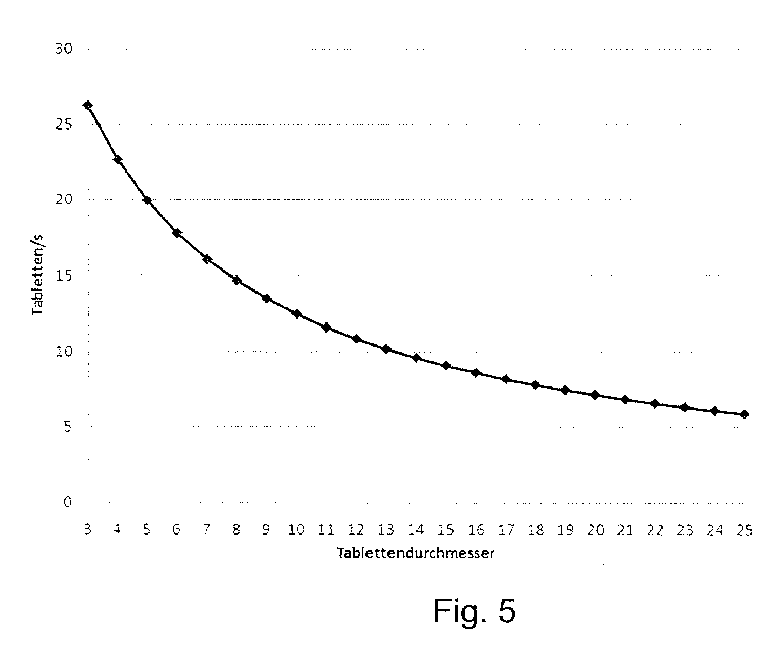

In Fig. 5 ist beispielhaft die erreichbare Leistung bei einer Tablettengeschwindigkeit v von beispielsweise 0,17 m/s für verschiedene Tablettengrößen dargestellt. Man erkennt, dass bei steigendem Tablettendurchmesser die Anzahl von überprüften Tabletten 4 pro Sekunde abnimmt.

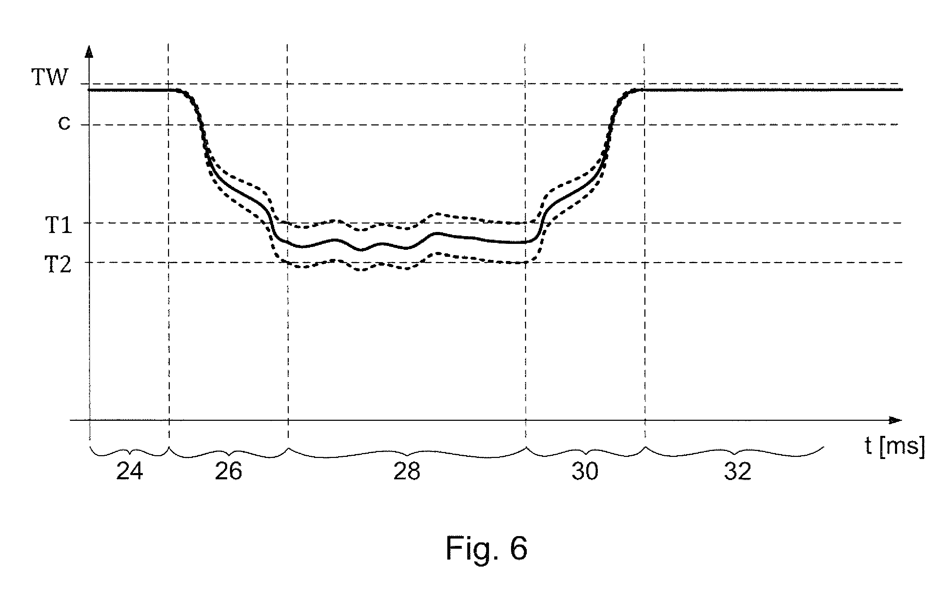

In Fig. 6 ist ein beispielhaft zu erwartender Signalverlauf der Ist-Messwerte des IR-Sensors 10 dargestellt. Zunächst misst der IR-Sensor 10 ein Signal, welches sich auf einem Plateau 24 bewegt und der konstanten Referenztemperatur TW des Wärmeelements 8 entspricht. Tritt die Tablette 4 in den Bereich des Messfeldes 14 ein, so nimmt die Signalintensität der vom IR-Sensor 10 detektierten IR-Strahlung entsprechend der zunehmenden Überdeckung des Wärmeelements 8 durch die Tablette 4 ab (Bereich 26). Sobald die Tablette 4 sich vollständig im Messfeld 14 befindet, wird ein Minimum bzw. unteres Plateau 28 des Signals des IR-Sensors 10 erreicht. Wenn die Tablette 4 das Messfeld 14 wieder verlässt, erfolgt wiederum ein Signalanstieg (Bereich 30) des vom IR-Sensor 10 empfangenen Signals. Anschließend geht der Ist-Messwert des IR-Sensors 10 erneut auf ein Plateau 32 über, welches der konstanten Temperatur des Wärmeelements 8 entspricht.

Es existieren nun verschiedene Aufnahme- und Auswertemöglichkeiten der Ist-Messwerte des IR-Sensors 10. In der Regel wird nur während der Zeit ausgewertet (Bereich 28), in der sich die Tablette 4 vollständig im Messfeld 14 befindet. Eine vorbestimmte Temperaturänderung bei Eintritt der Tablette 4 in das Messfeld 14 kann dabei, gegebenenfalls mit einer vorbestimmten Zeitverzögerung, als Triggersignal c verwendet werden. Dieses Triggersignal kann außerdem für eine Zählfunktion und mit vorbestimmter Zeitverzögerung für die Ausscheidefunktion genutzt werden.

Wie bereits zuvor erläutert ist das Messergebnis ein Temperaturdurchschnittswert über die gesamte Fläche des Messfeldes 14. Von dem in Fig. 6 dargestellten Graphen kann nun beispielsweise die minimale Signalhöhe im Bereich 28 oder das Integral unter dem Signal in einem Teil des Bereichs 28 oder im gesamten Bereich 28 als Entscheidungskriterium verwendet werden. Wenn die Ist-Messwerte außerhalb gewisser Toleranzwerte T1, T2 liegen, welche voreingestellt werden können, wird die entsprechende Tablette 4 ausgeschieden. Die Soll-Messwerte des Referenzsignals können über Testläufe mit einer guten Tablette 4 oder einer Folge guter Tabletten 4 erzeugt werden. Ebenso könnten entsprechende Soll-Messwerte per Software eingelernt werden.

Die Messung ist eine Relativmessung, so dass eventuelle Temperaturdriften keine Auswirkungen haben. Hierdurch kann auch beispielsweise eine leichte Verstaubung des IR-Sensors 10 kompensiert werden. Auch kann durch entsprechendes Nachregeln der Temperatur des Wärmeelements 8 die Temperaturdifferenz zwischen Tablette 4 und Wärmeelement 8 konstant gehalten werden, wenn sich beispielsweise die Temperaturen von Tabletten 4 oder Führungseinrichtung 2 bei Dauerbetrieb ändern.

Um sicherzustellen, dass sowohl Tabletten anderer Farbe als auch anderer Form sowie abgebrochene Tabletten sicher detektiert werden können, sollte das Flächenverhältnis des Messfeldes 14 zur Fläche der Tablette 4 im Bereich unterhalb von 3:1 liegen. Das Messfeld 14 sollte jedoch immer mindestens so groß wie die Tablette 4 selbst sein.

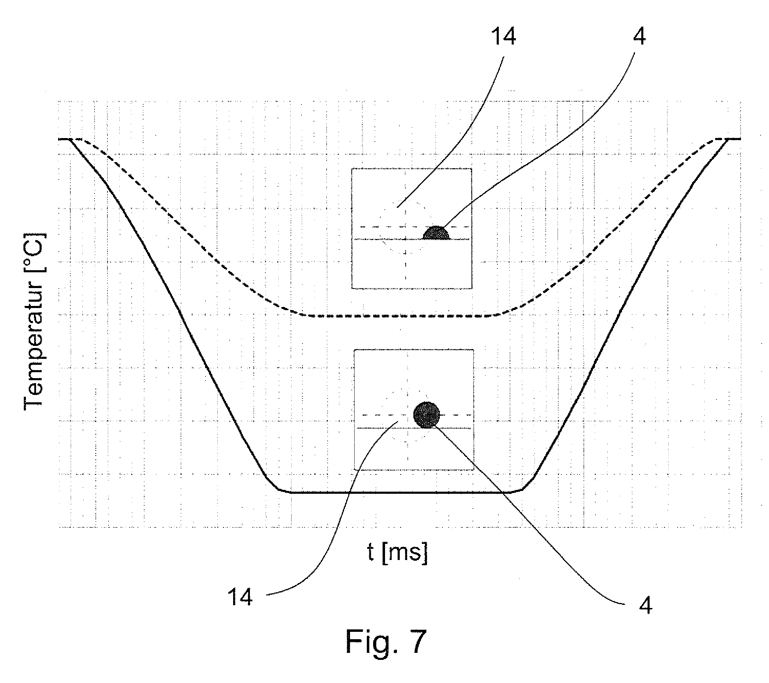

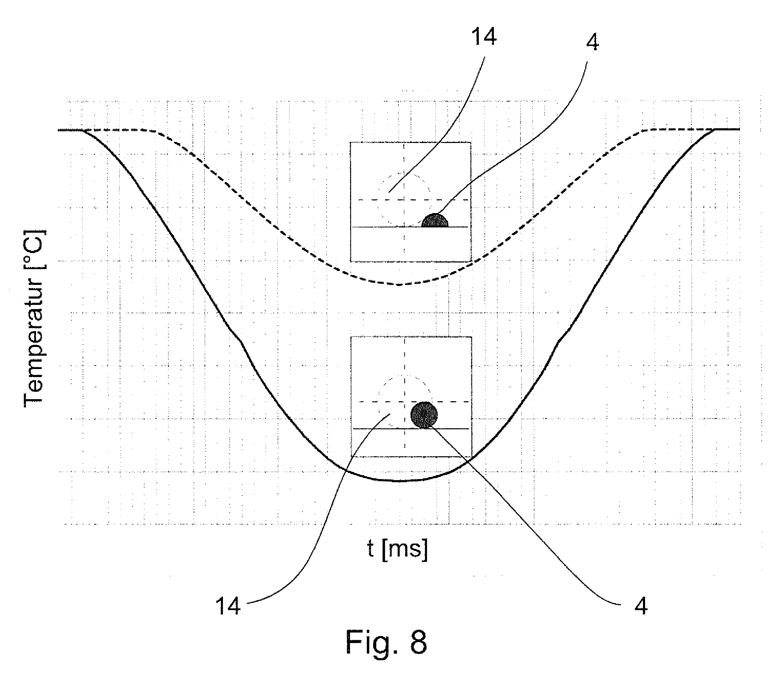

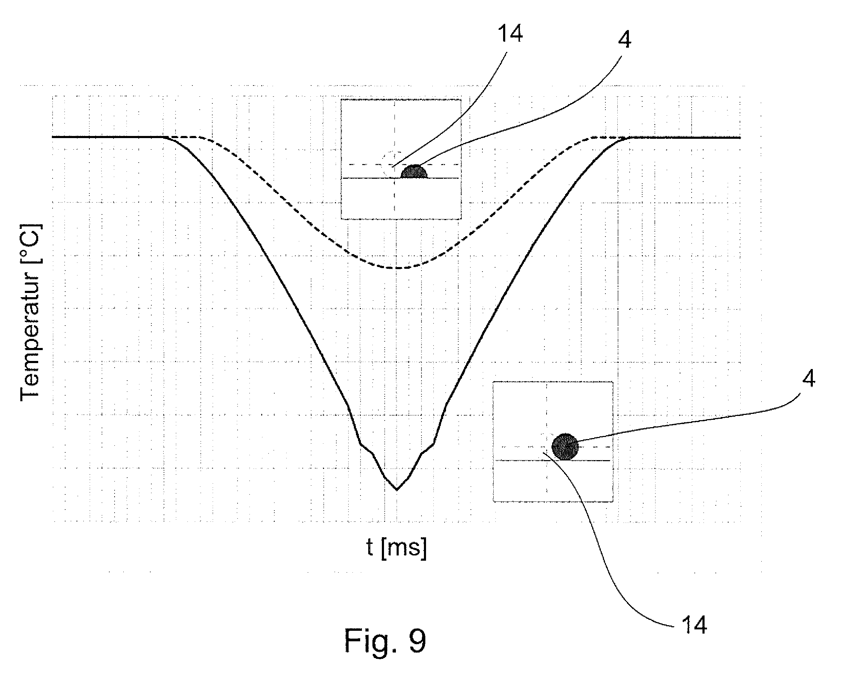

In Fig. 7 bis 9 sind einige theoretische Messkurven des IR-Sensors 10 für unterschiedliche Konfigurationen dargestellt. Wie in der Abbildung im rechten Teil der Figuren anschaulich dargestellt, ist das Messfeld 14 in Fig. 7 und 8 etwa 2,5-mal so groß wie die Fläche der Tablette 4. Die durchgezogene Linie gibt dabei den theoretisch zu erwartenden Verlauf der Ist-Messwerte des IR-Sensors 10 für eine intakte Tablette 4 an, während die gestrichelt gezeichnete Kurve die theoretisch zu erwartenden Ist-Messwerte für eine halbe Tablette darstellt. Selbiges gilt für Fig. 8, wobei die Tabletten 4 lediglich durch den unteren Bereich des Messfeldes 14 laufen und somit die Länge des Minimums der Temperaturmesskurve reduziert wird. In Fig. 9 ist die Situation dargestellt, dass das Messfeld 14 der Größe der Tablette 4 entspricht. Hier erkennt man, dass der Unterschied zwischen den Signalhöhen bei intakter Tablette und halber Tablette größer ist als in den beiden vorangegangenen Zeichnungen, allerdings die Kurven auch relativ spitze Minima aufweisen.

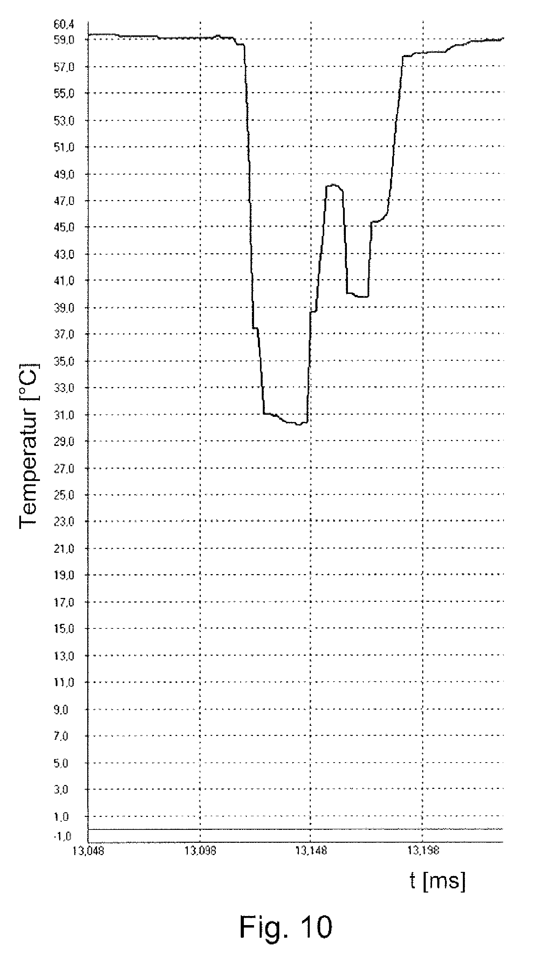

In Fig. 10 ist eine tatsächlich gemessene Temperaturkurve dargestellt, wobei das erste Minimum eine intakte Tablette 4 kennzeichnet und das zweite Minimum eine abgebrochene, halbe Tablette 4 kennzeichnet. Deutlich ist der Unterschied der Ausschläge sowohl hinsichtlich der Signalhöhe als auch hinsichtlich der Länge des Minimum-Plateaus ersichtlich.

Bei der Ausführung der Führungseinrichtung 2 als Vibrationsrinne (Fig. 2) kann die Geschwindigkeit der Tabletten 4 entlang des Tablettenpfades A im Wesentlichen konstant eingestellt werden, wodurch die Überprüfung erleichtert wird, während die Geschwindigkeit der Tabletten 4 entlang dem Tablettenpfad A bei der Ausführungsform aus Fig. 3 beim Durchlauf der Produktrutsche etwas zunimmt. Umgekehrt erfolgt bei der Ausführungsform gemäß Fig. 2 eine durch die Vibration der Vibrationsrinne hervorgerufene Zitterbewegung der Tabletten 4, was wiederum gewisse Messungenauigkeiten mit sich bringen kann. Dies ist bei der Ausführungsform gemäß Fig. 3 ausgeschlossen. Alle genannten Variationen beeinflussen jedoch das Ergebnis der erfindungsgemäßen Messung aufgrund deren Robustheit kaum und können bei der Grenzwerteinstellung der Toleranzen für gute Tabletten berücksichtigt werden.

Mit der erfindungsgemäßen Vorrichtung können die meisten gebrochenen Tabletten, Tabletten falscher Farbe und Tabletten falscher Form aufgefunden werden. Außerdem eignet sie sich für die Zählung der Tabletten. Bei mehrbahnigen Führungseinrichtungen 2 soll für jede Bahn der Führungseinrichtung 2 ein eigener IR-Sensor 10 vorgesehen sein.

Damit kann unmittelbar vor der Abfüllung der Tabletten 4 in die zugehörigen Flaschen bzw. Behälter eine sichere und robuste Überprüfung und Zählung der Tabletten durchgeführt werden.

## Patentansprüche

1. Vorrichtung zum Überprüfen von vereinzelten Tabletten (4) oder Kapseln mit:
einer Führungseinrichtung (2) für die vereinzelten Tabletten (4) oder Kapseln, die einen vorbestimmten Tablettenpfad (A) definiert,
einem IR-Sensor (10) zur Detektion von Strahlung im Infrarotbereich, und
einer Auswerteeinheit (16), die dazu geeignet ist, Ist-Messwerte des IR-Sensors (10) mit mindestens einem vorbestimmten Soll-Messwert zu vergleichen,**dadurch gekennzeichnet, dass**
im Bereich der Führungseinrichtung (2) ein als Wärmeplatte ausgebildetes Wärmeelement (8) angeordnet ist und
der IR-Sensor dem Wärmeelement (8) gegenüberliegt, wobei der Tablettenpfad (A) zwischen dem Wärmeelement (8) und dem IR-Sensor (10) verläuft und die Ist-Messwerte des IR-Sensors (10) Temperaturmesswerte sind.

2. Vorrichtung nach Anspruch 1, **dadurch gekennzeichnet, dass** das Wärmeelement (8) von der Führungseinrichtung (2) entkoppelt ist.

3. Vorrichtung nach Anspruch 1 oder 2, **dadurch gekennzeichnet, dass** das Wärmeelement (8) bezüglich seiner Temperatur einstellbar ist.

4. Vorrichtung nach einem der vorangehenden Ansprüche, **dadurch gekennzeichnet, dass** das Wärmeelement (8) aus Metall, vorzugsweise aus Aluminium, gebildet ist.

5. Vorrichtung nach einem der vorangehenden Ansprüche, **dadurch gekennzeichnet, dass** das Wärmeelement (8) auf einer dem Tablettenpfad (A) zugewandten Fläche mit einer nichtmetallischen Beschichtung mit hohem Emissionswert überzogen ist.

6. Vorrichtung nach einem der vorangehenden Ansprüche, **dadurch gekennzeichnet, dass** zwischen Wärmeelement (8) und Tablettenpfad (A) eine IR-durchlässige Platte (20), vorzugsweise aus IR-durchlässigem Glas, angeordnet ist.

7. Vorrichtung nach einem der vorangehenden Ansprüche, **dadurch gekennzeichnet, dass** zwischen Wärmeelement (8) und Tablettenpfad (A) eine Messfeldblende (22) angeordnet ist.

8. Vorrichtung nach einem der vorangehenden Ansprüche, **dadurch gekennzeichnet, dass** der IR-Sensor (10) eine Vorsatzlinse (12) zum Einstellen der Größe des Messfeldes (14) aufweist.

9. Vorrichtung nach einem der vorangehenden Ansprüche, **dadurch gekennzeichnet, dass** der Abstand zwischen IR-Sensor (10) und Messfeld (14) 20 bis 50 mm beträgt.

10. Vorrichtung nach einem der vorangehenden Ansprüche, **dadurch gekennzeichnet, dass** das Messfeld (14) eine variable Größe von 25 bis 1000 mm² aufweist.

11. Vorrichtung nach Anspruch 1, **dadurch gekennzeichnet, dass** die Ist-Messwerte des IR-Sensors (10) eine Temperaturkurve bilden.

12. Vorrichtung nach einem der vorangehenden Ansprüche, **dadurch gekennzeichnet, dass** die Führungseinrichtung (2) eine Vibrationsrinne ist.

13. Vorrichtung nach einem der vorangehenden Ansprüche, **dadurch gekennzeichnet, dass** die Führungseinrichtung (2) eine Produktrutsche ist.

## Claims

1. A device for inspecting individual tablets (4) or capsules comprising:
a guide device (2) for the individual tablets (4) or capsules, which defines a predetermined tablet pathway (A), an IR sensor (10) for detecting radiation in the infrared range, and
an evaluation unit (16), which is adapted to compare actual measurement values of the IR sensor (10) with at least one predetermined nominal measurement value, **characterized in that**
a heating element (8) designed as a hotplate is arranged in an area of the guide device (2), and
the IR sensor is arranged opposite the heating element (8), wherein the tablet pathway (A) passes between the heating element (8) and the IR sensor (10) and the actual measurement values of the IR sensor (10) are temperature measurement values.

2. The device according to Claim 1, **characterized in that** the heating element (8) is decoupled from the guide device (2).

3. The device according to Claim 1 or 2, **characterized in that** the heating element (8) is adjustable with respect to its temperature.

4. The device according to any one of the preceding claims, **characterized in that** the heating element (8) is made of metal, preferably of aluminum.

5. The device according to any one of the preceding claims, **characterized in that** on a surface facing the tablet pathway (A), the heating element (8) is covered with a nonmetallic coating having a high emission value.

6. The device according to any one of the preceding claims, **characterized in that** an IR-permeable plate (20), preferably made of IR-permeable glass, is arranged between the heating element (8) and the tablet pathway (A).

7. The device according to any one of the preceding claims, **characterized in that** a field-of-view diaphragm (22) is arranged between the heating element (8) and the tablet pathway (A).

8. The device according to any one of the preceding claims, **characterized in that** the IR sensor (10) includes an ancillary lens (12) for adjusting the size of the field of view (14).

9. The device according to any one of the preceding claims, **characterized in that** the distance between the IR sensor (10) and the field of view (14) is 20 to 50 mm.

10. The device according to any one of the preceding claims, **characterized in that** the field of view (14) has a variable size of between 25 to 1000 mm².

11. The device according to Claim 1, **characterized in that** the actual measurement values of the IR sensor (10) form a temperature curve.

12. The device according to any one of the preceding claims, **characterized in that** the guide device (2) is a vibrating trough.

13. The device according to any one of the preceding claims, **characterized in that** the guide device (2) is a product chute.

## Revendications

1. Dispositif permettant d'inspecter des comprimés (4) individuels ou des capsules comprenant :
un dispositif de guidage (2) pour les comprimés individuels (4) ou les capsules, qui détermine un trajet de comprimé prédéterminé (A),
un capteur à IR (10) pour détecter le rayonnement dans la gamme infrarouge, et
une unité d'évaluation (16), qui est adaptée pour comparer des valeurs de mesure réelles du capteur IR (10) avec au moins une valeur de mesure nominale prédéterminée,
**caractérisée en ce**
**qu'**un élément de chauffage (8) conçu comme une plaque chaude est agencée dans une zone du dispositif de guidage (2), et
**que** le capteur IR est agencé à l'opposé de l'élément chauffant (8), dans lequel le trajet de comprimé (A) passe entre l'élément chauffant (8) et le capteur IR (10) et les valeurs de mesure réelles du capteur IR (10) sont des valeurs de mesure de la température.

2. Dispositif selon la revendication 1, **caractérisé en ce que** l'élément chauffant (8) est découplé à partir du dispositif de guidage (2).

3. Dispositif selon la revendication 1 ou la revendication 2, **caractérisé en ce que** l'élément chauffant (8) est ajustable au niveau de sa température.

4. Dispositif selon l'une quelconque des revendications précédentes, **caractérisé en ce que** l'élément chauffant (8) est fait en métal, de préférence de l'aluminium.

5. Dispositif selon l'une quelconque des revendications précédentes, **caractérisé en ce que** sur la surface faisant face au trajet de comprimé (A), l'élément chauffant (8) est recouvert d'un revêtement non-métallique ayant une valeur d'émission élevée.

6. Dispositif selon l'une quelconque des revendications précédentes, **caractérisé en ce que** la plaque perméable à l'IR (20), de préférence fait d'un verre perméable à l'IR, est agencé entre l'élément chauffant (8) et le trajet de comprimé (A).

7. Dispositif selon l'une quelconque des revendications précédentes, **caractérisé en ce qu'**un diaphragme de champ de vision (22), est agencé entre l'élément chauffant (8) et le trajet de comprimé (A).

8. Dispositif selon l'une quelconque des revendications précédentes, **caractérisé en ce que** le capteur IR (10) comprend une lentille auxiliaire (12) permettant d'ajuster la taille du champ de vision (14).

9. Dispositif selon l'une quelconque des revendications précédentes, **caractérisé en ce que** la distance entre le capteur IR (10) et le champ de vision (14) est de 20 à 50 mm.

10. Dispositif selon l'une quelconque des revendications précédentes, **caractérisé en ce que** le champ de vision (14) a une taille variable entre 25 à 1000 mm².

11. Dispositif selon la revendication 1, **caractérisé en ce que** les valeurs de mesure réelles du capteur IR (10) forment une courbe de température.

12. Dispositif selon l'une quelconque des revendications précédentes, **caractérisé en ce que** le dispositif de guidage (2) est une cuve d'oscillation.

13. Dispositif selon l'une quelconque des revendications précédentes, **caractérisé en ce que** le dispositif de guidage (2) est une gouttière de produit.
